# EUROPEAN PATENT APPLICATION

(11) **EP 4 830 620 A1**
(43) Date of publication of application: **28.10.2026**
(21) Application number: 25382406.4
(22) Date of filing: 23.04.2025
(51) Int. Cl.: A61B 5/08, A61B 5/00, A61B 5/113

(54) **SYSTEM AND METHOD FOR MEASURING THE RESPIRATORY RATE IN DOGS**

(71) Applicant: AcuPet Health, S.L., 33005 Oviedo (ES)
(72) Inventor: ACUÑA ALVAREZ-URIA, Cristina, 33005 Oviedo (ES)
(74) Representative: Clarke, Modet y Cía., S.L.

(57) **Abstract**

Systems and methods for measuring the respiratory pattern in dogs. The system comprises a dog harness (2) with electrodes (4a, 4b) arranged in a chest region; a measuring device (3) fastened to the harness (2) and comprising capacitive sensors (12a, 12b) electrically connected to the electrodes (4a, 4b); and a data processing unit (14) configured for filtering the electrical capacity signals (31a, 31b) from each capacitive sensor (12a, 12b), detecting local peaks (35) or valleys (37) in each filtered electrical capacity signal (33a, 33b), calculating, for each filtered electrical capacity signal (33a, 33b), an average temporal distance (36) between the detected peaks (35) or valleys (37), determining a respiratory rate in each filtered electrical capacity signal (33a, 33b) using the corresponding average temporal distance (36), and determining a respiratory rate (40) of the dog (6) from the respiratory rates of the filtered electrical capacity signals (33a, 33b).

## Description

### FIELD OF THE INVENTION

The present invention falls within the field of systems and methods for detecting the respiratory pattern in animals and, in particular, canine pets in a non-invasive manner.

### BACKGROUND OF THE INVENTION

Currently, different techniques are known for obtaining the respiratory rate pattern in canine pets in a non-invasive way:
- Thoracic bioimpedance (pneumographic impedance): It consists of placing electrodes in the body of the animal and passing a small alternating current through the thorax to detect changes in its electrical impedance due to breathing. As the dog inhales and exhales, the electrical resistance between the electrodes varies in synchrony with the air inlet/outlet, and the equipment records that oscillating signal. Bioimpedance allows the respiratory rate to be extracted and the depth of each breath to be estimated.
- Electrical Impedance Tomography (EIT): It is a more advanced technique that employs multiple electrodes distributed around the chest to obtain a dynamic image of pulmonary ventilation. A small alternating current is applied by pairs of electrodes and the voltage on the others is measured. Using algorithms, it is reconstructed how the electrical impedance changes in different lung regions with each breath. Since air in the lungs alters local conductivity (air increases impedance to where it accumulates), the system generates real-time ventilation maps, showing which areas of the dog's lung are filled with air and to what degree. The EIT converts the electrical variations produced by breathing into functional images, helping veterinarians to visualize problems such as atelectasis or uneven ventilation in a non-invasive way.
- Accelerometry: It is a technique that uses accelerometers to detect respiratory movements in animals. The document "Validation of a Wearable System for Respiratory Rate Monitoring in Dogs" (Angelucci et al., IEEE Journal, 2024) discloses a portable system that allows estimating the respiratory rate in dogs using 3 inertial sensors (IMUs).
- UWB Radar: The document "Non-Contact Vital Signs Monitoring of Dog and Cat Using a UWB Radar" (Wang et al., Animals, 2020) discloses the use of ultra-wideband (UWB) radar to monitor the respiratory rate of dogs in a non-invasive manner.

Several of these techniques take advantage of impedance or electric field changes during the respiratory cycle to monitor the ventilatory pattern in a non-invasive way. The present invention presents a solution based on capacitive sensors that detect changes in the electric field or capacitance around the body caused by respiratory movement.

### DESCRIPTION OF THE INVENTION

The invention relates to a system and a method for measuring the respiratory rate in dogs in a non-invasive way, which uses a harness or capacitive belt as a portable monitor to remotely monitor the breathing of a dog, which may have lung problems, detecting in real time if the pattern becomes irregular or if ventilation decreases. In the present invention, two capacitive sensors, preferably identical, are used, placed on both sides of the pet's chest and which inject alternating current of very low levels, imperceptible to the animal, to obtain more coherent and independent values of the animal's movements during the captures.

Physically, a dog's body can be modeled as a set of conductors (tissues, blood) and dielectrics (cell membranes, air in the lungs) that determine its total electrical impedance. Impedance includes both resistance (current flow through ionic liquids) and capacitive reactance (charge storage in membranes and interfaces). During breathing, structural changes in the thorax alter these electrical properties. A key factor is the poor conductivity of air compared to that of wet tissues: air is essentially an insulator, while body fluids (with high water and ion content) are good conductors. Therefore, by inhaling and filling the lungs with air, the overall conductivity of the thorax decreases and its electrical impedance increases. Conversely, when exhaling air and contracting the lungs, the volume of air in the chest drops and the proportion of fluid tissue increases, which reduces impedance. In other words, breathing constantly modulates the body's electrical resistance: more air in the lung means more resistance (less current passes), less air means less resistance (more current passes). These changes occur in each respiratory cycle, so the impedance goes up and down following the same rate as breathing.

In this way, inspiration equals lower capacitance (higher impedance) and exhalation equals higher capacitance (lower impedance) in the body circuit. An almost linear close correlation has been observed between the impedance changes and the volume of air breathed, so that by measuring the impedance, the amount of air in the lungs at each moment can be estimated.

These electrical variations present the same periodic pattern as breathing. The capacitance/impedance of the body oscillates synchronously with each inhalation and exhalation. This means that, by representing the impedance or capacitance of the body over time, a wave is obtained that rises and falls in phase with the air inlet and outlet. In a quietly breathing dog, each peak of the electrical signal corresponds to one inhalation (lungs filled with air, maximum impedance) and each valley corresponds to one exhalation (lungs emptied, minimum impedance). Thus the electrical dynamics of the body remixes the respiratory cycle in terms of electrical signals, allowing to identify the breathing pattern from electrical measurements.

Therefore, the present invention takes advantage of the fact that the electrical signal changes the breathing compass, so that the pattern of the body's electrical capacity is a mirror of the respiratory pattern. The invention employs capacitive sensors that function as capacitor plates that may be in contact with the skin or at close range. When the dog breathes, the displacement of its thorax and the variation in the amount of (dielectric) air modulate the capacitance between the body and the capacitive sensor.

In particular, the system for measuring respiratory rate in dogs of the present invention comprises a dog harness and a measuring device attached to the harness. The harness includes at least one electrode arranged in a chest region, each electrode being electrically connected to a respective metal contact. The harness, which normally has two leashes, can be implemented by means of a belt with a single strap that adapts to the thoracic region of the dog.

The measurement device comprises a housing, at least one battery, a data processing unit, a fastening system configured to fasten the housing to the harness, and at least one capacitive sensor.

Each capacitive sensor is electrically connected to a corresponding metal contact of the harness when the measuring device is fastened to the harness, and is responsible for making electrical capacity measurements.

The data processing unit is configured to receive an electrical capacity signal from each capacitive sensor, wherein each electrical capacity signal includes a plurality of electrical capacity measurements performed by the corresponding capacitive sensor during a sampling time when the system is in use, i.e. when the harness is placed on a dog and the measurement device is fastened to the harness.

The data processing unit is responsible for filtering each electrical capacity signal, detecting local peaks (or valleys) in each filtered electrical capacity signal, calculating a average temporal distance between the detected peaks (or valleys), and determining a respiratory rate associated with each filtered electrical capacity signal as a function of the corresponding average temporal distance between peaks/valleys. The data processing unit is ultimately responsible for determining a respiratory rate of the dog from the respiratory rate associated with each filtered electrical capacity signal. These processes of capturing and analyzing data from the capacitive sensors are repeated iteratively, so that the data processing unit is able to continuously monitor the respiratory rate of the dog.

A second aspect of the present invention relates to a method for measuring respiratory rate in dogs comprising the following steps: placing in a dog a harness including at least one electrode arranged in a chest region, each electrode being electrically connected to a respective metal contact; fastening to the harness a measuring device including at least one capacitive sensor, such that each capacitive sensor is electrically connected to a corresponding metal contact of the harness; receiving an electrical capacity signal from each capacitive sensor, where each electrical capacity signal includes a plurality of electrical capacity measurements performed by the corresponding capacitive sensor during a sampling time; filtering each electrical capacity signal; detecting local peaks or valleys in each filtered electrical capacity signal; calculating an average temporal distance between the detected peaks or valleys; determining (a respiratory rate associated with each filtered electrical capacity signal based on the corresponding average temporal distance between peaks or valleys; and determining a respiratory rate of the dog from the respiratory rate associated with each filtered electrical capacity signal.

### BRIEF DESCRIPTION OF THE FIGURES

Next, a series of drawings that help to better understand the invention and that are expressly related to an embodiment of said invention that is presented as a non-limiting example of said invention are very briefly described below.
Figures 1A and 1B show a system for measuring respiratory rate in dogs according to an embodiment.
Figures 2A and 2B show the system mounted on a dog.
Figures 3A and 3B illustrate the measuring device according to an embodiment.
Figure 4 depicts a fastening system employed to couple the measuring device to the harness.
Figure 5 shows an exploded view of the components of the measuring device.
Figure 6 shows the reception and processing, by the data processing unit, of the electrical capacity signals from the capacitive sensors.
Figure 7 illustrates electrical capacity signals received by the data processing unit during a sampling time.
Figure 8 depicts filtered electrical capacity signals.
Figure 9 illustrates the local peaks detected in the filtered electrical capacity signals of Figure 8.
Figure 10 is a flow diagram of a method for measuring respiratory rate in dogs according to an embodiment.
Figure 11 shows the capacitance distribution in a system that employs two capacitive touch sensors.
Figure 12 is a graph of the signals from a capacitive touch sensor.
Figure 13 is a graph of the pulse counter of the output signal of the capacitive touch sensor, where the captures of the moments of exhalation (expulsion of air) and inhalation (aspiration of air) of the pet can be observed.
Figure 14 illustrates a flowchart of a process of calculating respiratory rate using two capacitive sensors, in accordance with one embodiment.

### DETAILED DESCRIPTION OF THE INVENTION

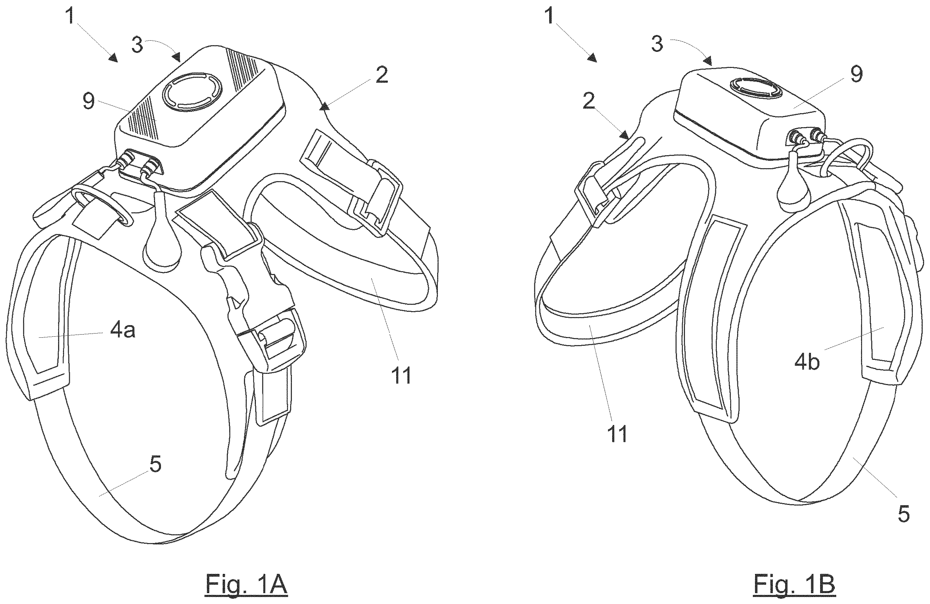

The present invention relates to a system 1 for measuring the respiratory rate in dogs comprising, as shown in the embodiment of Figures 1A and 1B, a dog harness 2 and a measuring device 3 which, in use, is mounted on the harness 2.

The harness 2 includes at least one electrode (4a, 4b) arranged in a chest region of the harness 2, i.e. in a position such that the at least one electrode (4a, 4b) is adapted to contact the thorax of a dog when the harness 2 is placed on the dog. In the embodiment shown in Figures 1A and 1B the harness comprises two metal electrodes (a first electrode 4a located on the left side of the harness and a second electrode 4b on the right side), implemented by respective metal sheets located on each side of a rear strap 5 of the harness (opposite to a front strap 11) responsible for surrounding the thorax of a dog, arranging the electrodes in a curved shape to adapt to both sides of the thorax of the animal. Two sensors are preferably used in order to have two samples and thus be able to better filter the intrinsic errors inherent in working with animals, such as sudden movements and blows to the device, among others. In addition, and although the information obtained from both sensors should be similar in healthy animals, if different values are obtained, some type of pathology can be detected in the pet.

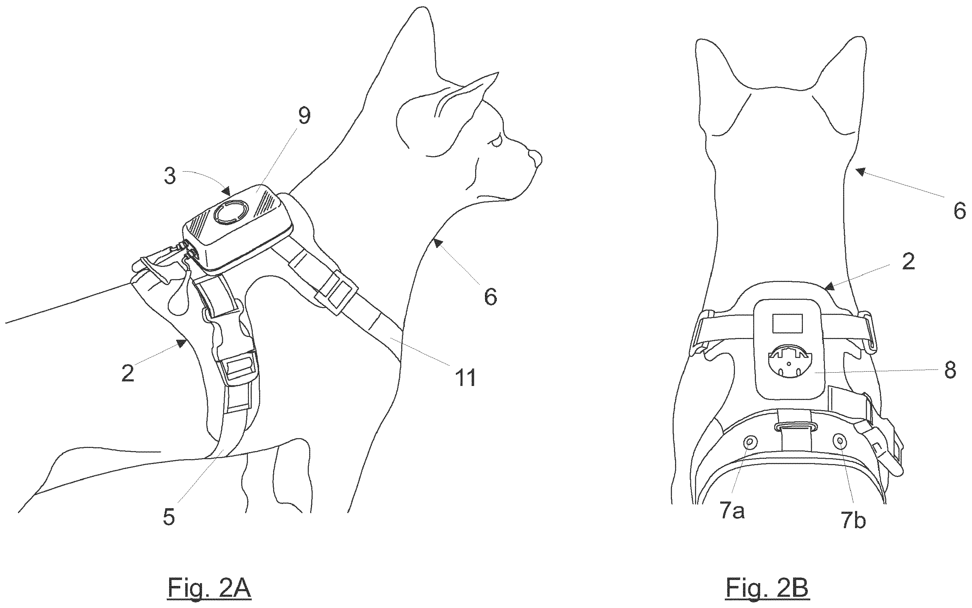

Figures 2A and 2B show the harness 2 of the previous figures placed on a dog 6. Each electrode (4a, 4b) is electrically connected to a respective metal contact (a first metal contact 7a and a second metal contact 7b), as illustrated in figure 2B, in which the measuring device 3 is not placed on the harness 2 (only a support 8 used to mount the measuring device 3 on the harness 2 is shown). In said figure 2B the first electrode 4a (corresponding to the left pectoral loop) is in electrical contact with the first metal contact 7a and the second electrode 4b (corresponding to the right pectoral loop) is connected to the second metal contact 7b.

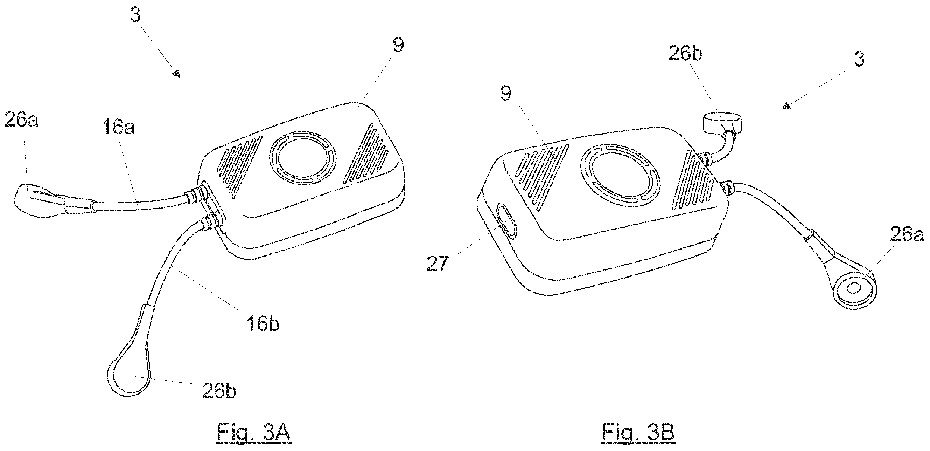

Figures 3A and 3B show in different views the measuring device 3 according to the embodiment of the previous figures, which comprises a housing 9 from which external cables (16a, 16b) come out with respective connectors (26a, 26b) at their ends adapted to engage and contact the metal contacts (7a, 7b), once the measuring device 3 has been mounted on the harness 2. Inside the housing 9, different electronic components of the measuring device 3 are installed, which are powered by a rechargeable battery through a charging port 27 (e.g., a USB C port).

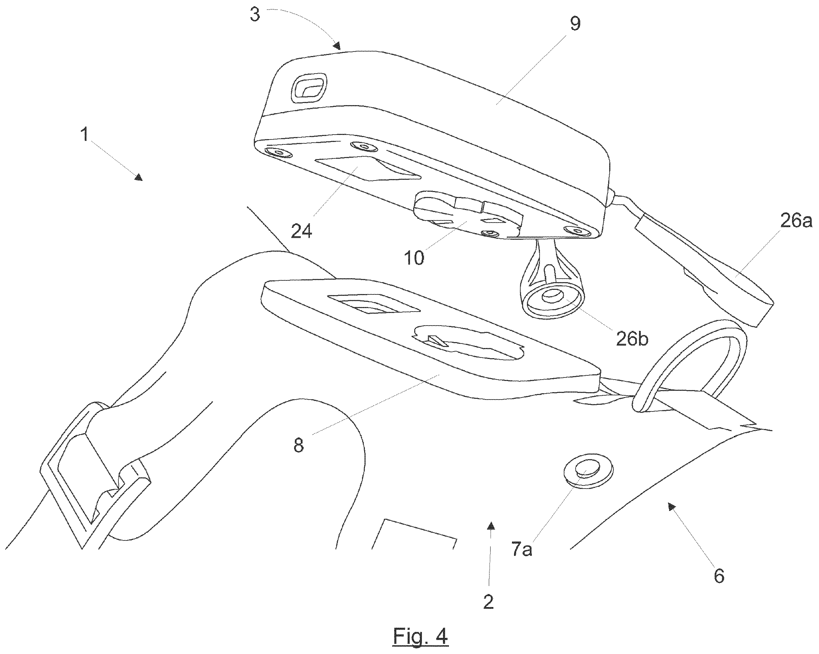

The measuring device 3 is preferably mountable and detachable to the harness through a fastening system, although it may be permanently fastened to the harness using any fastening means (e.g. by sewing, adhesive, etc.). In the embodiment of figure 4, the fixing system 10 comprises a quarter-turn locking system that fits into a complementary opening made in the support 8 attached to the harness 2, although any fixing system capable of fixing the housing 9 to the harness 2 can be used.

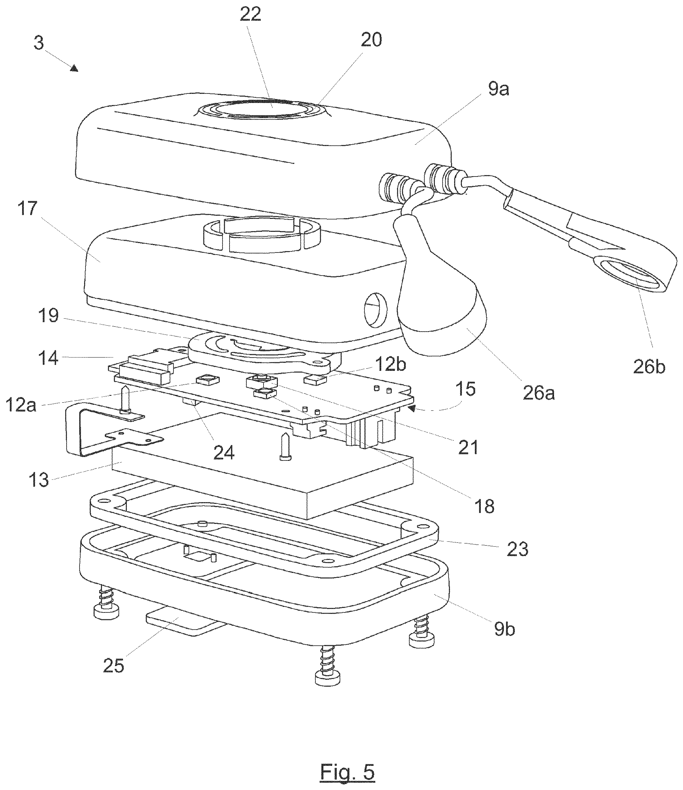

In figure 5 is illustrated, according to a possible embodiment, an exploded view of the components integrated into the housing 9 of the measuring device 3 (formed by an upper housing 9a and a lower housing 9b), including at least one capacitive sensor (12a, 12b) configured to perform electrical capacity measurements, a battery 13 for powering the electronic components and a data processing unit 14. In this embodiment the measuring device 3 comprises a first capacitive sensor 12a and a second capacitive sensor 12b, each capacitive sensor (12a, 12b) being electrically connected to a corresponding metal contact (7a, 7b) of the harness 2 when the measuring device 3 is fixed to the harness 2. In this way, the first capacitive sensor 12a is responsible for measuring the electrical capacity on the left side of the thorax of the animal and the second capacitive sensor 12b on the right side of the thorax. In this embodiment the capacitive sensors (12a, 12b) contact the metal contacts (7a, 7b) through the connectors (26a, 26b) located at the ends of the external cables (16a, 16b). In other embodiments such external cables (16a, 16b) could be dispensed with; for example, the connectors (26a, 26b) could be located at the base of the housing 9 and adapted to directly contact the metal contacts (7a, 7b) when the housing 9 is fixed to the harness 2. The data processing unit 14 and the capacitive sensors (12a, 12b) are preferably integrated into a same PCB 15.

The measuring device 3 can include other additional elements, for example:
- A translucent cover 17 that allows the passage of light, generated by a LED 18 and that crosses a light guide 19, towards an opening 20 made in the upper housing 9a. The led 18 is preferably an RGB color led, to provide color-dependent information about the status of the device and/or the status of the animal.
- A switch 21 for switching the device off and on, actuated by a pushbutton 22 in the upper housing 9a.
- A water tightness gasket 23.
- A temperature sensor 24 located on the PCB 15, configured to capture the temperature of the body of the dog 6 through an IR viewer 25 in the lower housing 9b.

Although in the embodiments of the above figures two electrodes (4a, 4b) are employed to contact both sides of the thorax of the dog 6, two metal contacts (7a, 7b), two connectors (26a, 26b) and two capacitive sensors (12a, 12b), in other embodiments a different number of these elements may be employed. For example, in one embodiment the system 1 may employ an electrode that contacts any region of the thorax of the dog 6, a metal contact, a connector, and a capacitive sensor.

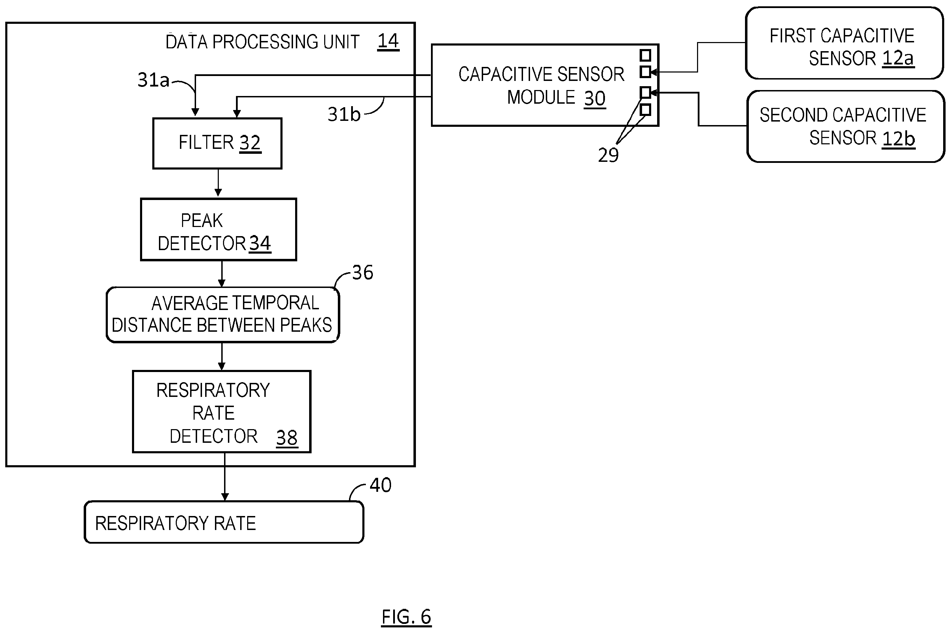

The data processing unit 14 is configured to receive an electrical capacity signal from each capacitive sensor (12a, 12b), wherein each electrical capacity signal includes a plurality of electrical capacity measurements performed by the corresponding capacitive sensor (12a, 12b) during a sampling time when the harness 2 is placed on a dog 6. Figure 6 shows the reception of the electrical capacity signals (first electrical capacity signal 31a and second electrical capacity signal 31b) from the capacitive sensors (12a, 12b) by the data processing unit 14. The system 1 may comprise a hardware module, capacitive sensor module 30, specifically designed to read changes in capacity at its different input ports 29. The capacitive sensor module 30 may be integrated into the data processing unit 14 itself or, as shown in figure 6, may be a module external to said data processing unit 14.

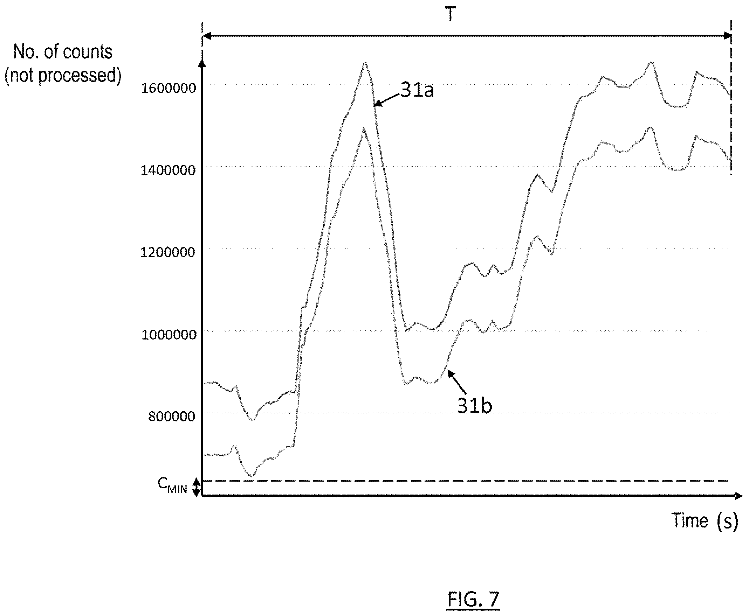

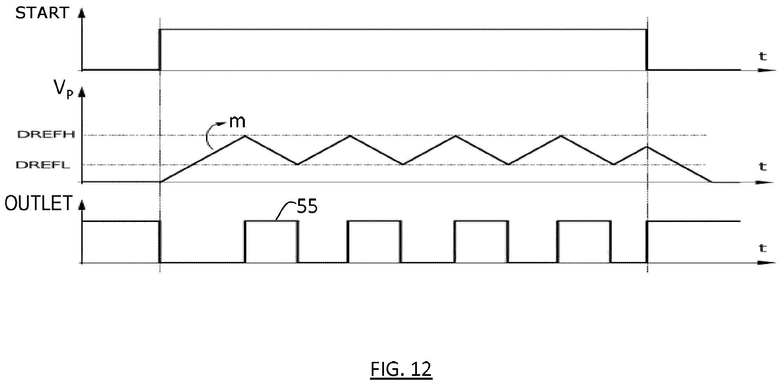

Figure 7 illustrates, by way of example, electrical capacity signals (31a, 31b) obtained by the capacitive sensor module 30, and provided to the data processing unit 14, during a sampling time T. In the embodiment shown in figure 7 the capacitive sensor module 30 provides a number of counts (which matches the number of output pulses 55 of the capacitive sensor shown in Figure 12), which is proportional to the rate of breathing. The electrical capacity measurements of each capacitive sensor can therefore be expressed in number of counts/pulses or in a unit of electrical capacity (e.g., pF). In the capacitive sensor module 30, the collection of samples can be scheduled for a certain fixed sampling time T or, alternatively, the collection of a certain number N of samples (which can take a variable sampling time T depending on the measured electrical capacity). To achieve good results, the electrical capacity signals (31a, 31b) collected by the capacitive sensors (12a, 12b) must be filtered and conditioned.

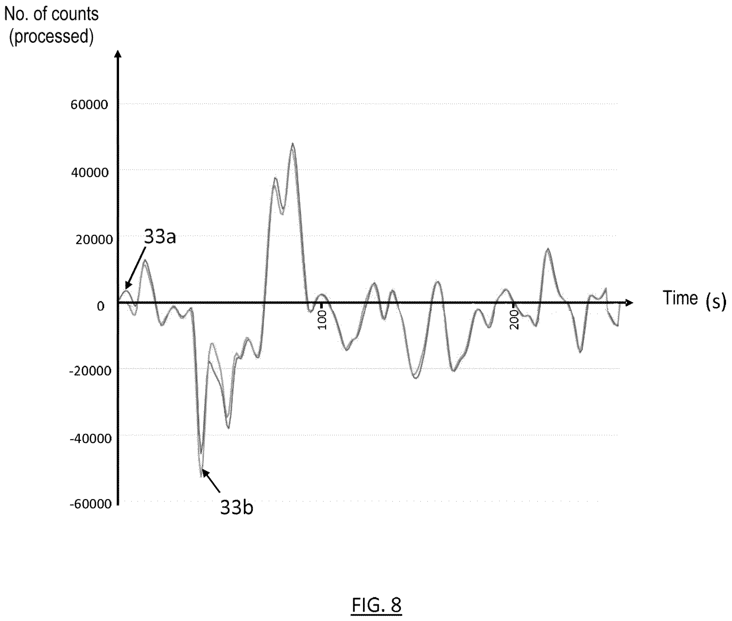

The data processing unit 14 is responsible for performing continuous readings of the capacity variations in the capacitive sensors (12a, 12b), converting them to digital values, and filtering noises from each electrical capacity signal (31a, 31b) received using a filter 32, which can be a software or hardware filter. Figure 8 represents, in an illustrative manner, filtered electrical capacity signals (first filtered electrical capacity signal 33a and second filtered electrical capacity signal 33b), from which their average value (the DC value of the signal) has also been removed. In this example both filtered signals, which respectively capture the electrical capacity on the left and right side of the thorax of the dog 6, substantially coincide.

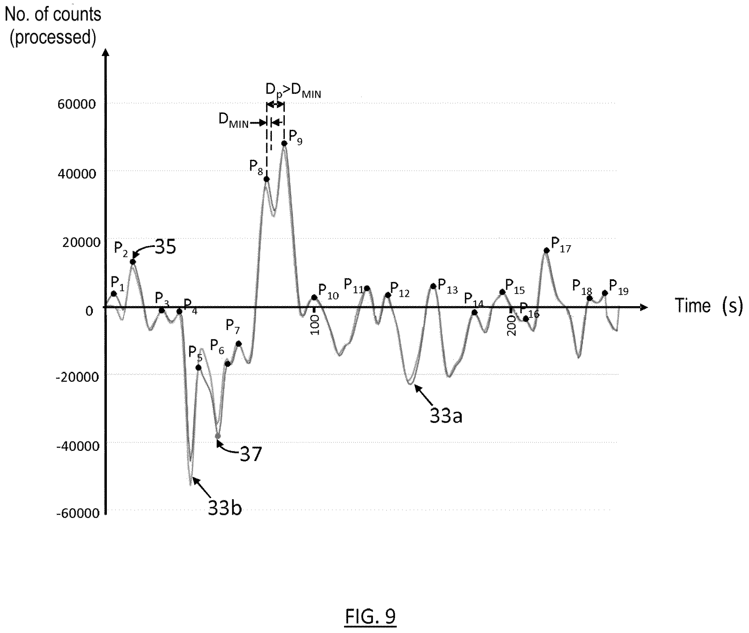

The data processing unit 14 is also configured to detect local peaks or valleys in each filtered electrical capacity signal (33a, 33b), using a peak detector 34, which is preferably implemented by an algorithm or software module of the data processing unit 14. The detected peaks or valleys are not the only absolute maximum or minimum of the signal at the sampling time, but maxima (peaks) or minima (valleys) in local signal environments. Figure 9 illustrates the detection of local peaks 35 {P1, P2,..., P19} in the filtered electrical capacity signals, although local valleys 37 could be detected instead. For the detection of local peaks or valleys, any prior art algorithm for detecting peaks in digital signals can be used. For example, the different samples can be traversed incrementally and a sample counter increased while the capacity value of the samples is increasing, detecting a local peak in that sample in which a subsequent sample has a lower value.

As a further condition for the detection of the local peaks or valleys, it is preferably required that the average electrical capacity of the measurements exceeds, in absolute value, a predetermined threshold value C_{MIN} of electrical capacity (or, equivalently, a threshold value of number of counts of the capacitive sensor module 30). The threshold value C_{MIN} serves to detect if the harness 2 is placed on the dog 6 and the electrodes make contact with the chest of the animal. If the average value of the electrical capacity of the measurements is not greater than this threshold value C_{MIN}, all the measurements are discarded. In the example shown in figure 7, it is verified that the average of the electrical capacity measurements is higher than the threshold value C_{MIN}, therefore it is considered that the measurements are valid (the harness is placed on the dog 6), and they are not discarded. In one embodiment, the threshold value C_{MIN} is obtained by calculating the average value of the electrical capacity of the measurements without placing the harness 2 to the dog 6 and with the harness 2 (and the measuring device 3) placed. The threshold value C_{MIN} is determined as the average value of the electrical capacity of the measurements without placing the harness plus an additional value ("offset"), but without approaching the average value of the electrical capacity of the measurements with the harness placed. For example, if the average electrical capacity without placing the harness is 25 pF and the average with the harness placed is 2000 pF, a value of 100 pF can be considered as a threshold value, so that measurements that do not reach this average value are discarded. This can be done once only and no additional calibrations are required.

Likewise, for the detection of local peaks or valleys, a minimum temporary distance (or number of samples) between adjacent peaks or valleys may also be required, to avoid any noise that could not be corrected with the filtering of the signal. For example, if a maximum respiratory rate of 60 breaths per minute (i.e., one breath per second) is considered, which in no case can be exceeded, the minimum temporary distance between two adjacent local peaks or valleys should always be greater than 1 second. Figure 9 shows the check that the distance Dₚ between the peaks P₈ and P₉ must be greater than D_{MIN}. As in the depicted example said condition is met, both peaks are valid. If the distance between both peaks were less than DMIN, one of them would be discarded (e.g., P₉, if the check is made with increasing times, starting from P₁), since it would correspond to a spurious peak.

The data processing unit 14 is configured to determine, in each filtered electrical capacity signal (33a, 33b), a average temporal distance 36 between the detected local peaks 35 (or valleys 37). To this end, the data processing unit 14 determines the distance Dₚ between adjacent non-discarded peaks 35 (or valleys 37) (Figure 9 shows the distance Dₚ between peaks P₈ and P₉) and calculates the average of said distances Dₚ. The distance Dₚ between adjacent peaks can be expressed in time or in number of samples, as a function of the axis of abscissa of the graph of figure 9, which can be expressed in time or in number of samples. If the distance Dₚ between adjacent peaks (or valleys) is expressed in number of samples, the data processing unit 14 converts said distances Dₚ to a time scale for determining the time-average distance 36.

The data processing unit 14 then determines, using a respiratory rate detector 38 (algorithm or software module), a respiratory rate associated with each filtered electrical capacity signal (33a, 33b) as a function of the corresponding average temporal distance 36 between local peaks 35 (or valleys 37). If, for example, it is detected that the average temporal distance 36 between peaks for the first 33a and second 33b filtered electrical capacity signal is 5.8 seconds and 6.2 seconds respectively (which implies that on average there is a peak every 5.8 seconds and 6.2 seconds, respectively), the respiratory rate associated with the first filtered electrical capacity signal 33a would be 1 breath every 5.8 seconds, that is, 10.3 breaths per minute, while the respiratory rate associated with the second filtered electrical capacity signal 33b would be 1 breath every 6.2 seconds, that is, 9.7 breaths per minute.

From the respiratory rate associated with each filtered electrical capacity signal (33a, 33b), the respiratory rate detector 38 of the data processing unit 14 determines a respiratory rate 40 of the dog 6. The respiratory rate 40 of the dog 6 may be calculated as an average (or a weighted average, in case different weights are assigned to the captured electrical capacity signals) of the respiratory rates associated with the filtered electrical capacity signals (33a, 33b). In the example above the average of 10.3 and 9.7 breaths per minute would be 10 breaths per minute.

In one embodiment, the horizontal axis of figure 9 may represent numbers of samples (Sample 1, Sample 2, ..., Sample N) instead of time. When the capacitive sensor module 30 is activated, the data processing unit 14 starts counting time and captures a programmed number N of samples. When the last sample N is reached, the data processing unit 14 records the sampling time T invested in capturing the N samples. As the number N of total samples of the measurement and the total time spent in the measurement (sampling time T) are known, the data processing unit 14 can calculate the average time between samples (average sampling period).

Depending on the hardware used, the time that passes between two consecutive samples (sampling period) can be variable, in those cases in which the hardware only allows to program the number of samples to be taken, since sample acquisitions can take more or less depending on the capacity that is connected to the capacitive sensor. Subsequently, the data processing unit 14 would obtain the distance between peaks (in number of samples, rather than time), and averages all the distances between peaks. Finally, the data processing unit 14 would convert this average to respiratory rate per minute.

For example, if the data processing unit 14 is scheduled to take 1000 samples, and a sampling time T of 9.6 seconds is obtained, the average distance between samples would be 9.6 ms. If the data processing unit 14 detects that there is a peak every 600 samples, it calculates an average temporal distance 36 between the detected peaks 35 of 5.76 seconds. Thus, in the case that the data processing unit 14 operates with a number of programmed samples, and obtains a distance between peaks expressed in number of samples, said distance is transformed into a average temporal distance 36 (expressed in seconds, instead of samples) between the detected peaks/valleys. The calculated respiratory rate would be equivalent to the inverse of the average temporal distance 36, which in this example would be 1 breath every 5.76 seconds (i.e., 10.4 breaths per minute).

The calculated respiratory rate 40 may be sent, via a wireless communication module (e.g., Wi-Fi, Bluetooth), to an external device, such as a remote server. Depending on the value of the respiratory rate 40, the data processing unit 14 can control the illumination (e.g., color and intensity) of the housing by activating the LED 18. The data processing unit 14 can also use the dog temperature information provided by the temperature sensor 24 to, in combination with the respiratory rate 40, activate one or the other color of the LED 15.

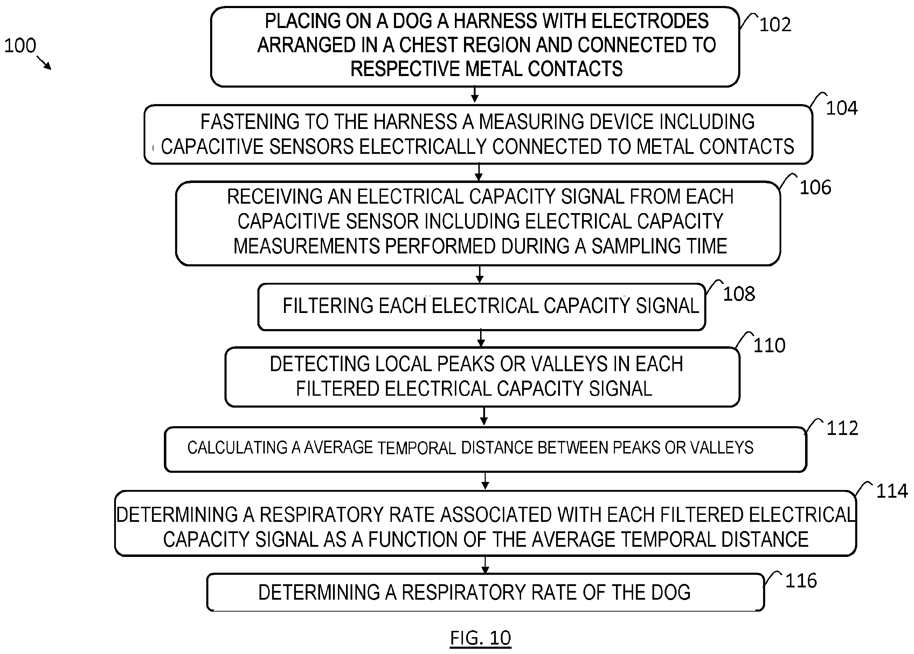

Figure 10 is a flow chart of a method 100 for measuring respiratory rate in dogs according to an embodiment of the present invention. The method 100 comprises the following steps:
- Placing 102 on a dog 6 a harness 2 including at least one electrode (4a, 4b) arranged in a chest region, each electrode (4a, 4b) being electrically connected to a respective metal contact (7a, 7b).
- Fastening 104 to the harness 2 a measuring device 3 including at least one capacitive sensor (12a, 12b), such that each capacitive sensor (12a, 12b) is electrically connected to a corresponding metal contact (7a, 7b) of the harness 2.
- Receiving 106 an electrical capacity signal (31a, 31b) from each capacitive sensor (12a, 12b), where each electrical capacity signal (31a, 31b) includes a plurality of electrical capacity measurements performed by the corresponding capacitive sensor (12a, 12b) during a sampling time T.
- Filtering 108 each electrical capacity signal (31a, 31b).
- Detecting 110 local peaks 35 or valleys 37 in each filtered electrical capacity signal (33a, 33b).
- Calculating 112 a average temporal distance 36 between the detected peaks 35 or valleys 37.
- Determining 114 a respiratory rate associated with each filtered electrical capacity signal (33a, 33b) as a function of the corresponding average temporal distance 36 between peaks 35 or valleys 37.
- Determining 116 a respiratory rate 40 of the dog 6 from the respiratory rate associated with each filtered electrical capacity signal (33a, 33b).

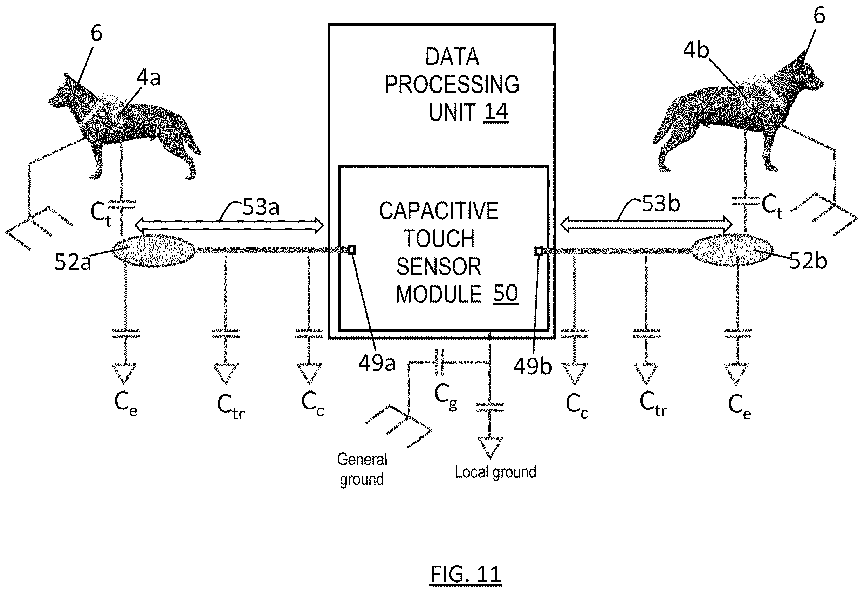

In one embodiment, each capacitive sensor (12a, 12b) is a capacitive touch sensor and the capacitive sensor module 30 is a capacitive touch sensor module. Figure 11 shows the capacitance distribution in a capacitive touch sensor system formed by a first capacitive touch sensor 52a and a second capacitive touch sensor 52b in electrical connection with the first electrode 4a (located on the left side of the thorax of the dog 6) and the second electrode 4b (located on the right side of the thorax of the dog 6), respectively. In the embodiment shown in figure 11 the capacitive touch sensor module 50 forms an integral part of the data processing unit 14, implemented for example in a system on chip (SoC) of the ESP32 family (e.g., ESP32-S3-MINI-1-N8), although it could be a module external to the data processing unit 14. The capacitive touch sensor module 50 measures, through each input port (49a, 49b), the total capacitance in each capacitive touch channel (a first left capacitive touch channel 53a, corresponding to the left loop of the thorax of the dog 6, and a second capacitive touch channel 53b for the right side of the thorax of the animal), which is composed of:
- C_{g}: Capacitance between the local ground of the circuit and the general ground.
- C_{c}: Intrinsic parasitic capacity of the PCB 15 of the data processing unit in which the capacitive touch sensor module 50 is integrated.
- Cₜᵣ: Parasitic capacitance between the trace and the local ground of the circuit.
- C_{w}: Capacitance of the cable joining the PCB 15 with the electrode (4a, 4b) of the harness 2.
- Cₑ: Parasitic capacitance between the electrode of the capacitive touch sensor (52a, 52b) and the ground of the circuit.
- Cₜ: Capacitance formed by the side of the chest of the dog 6 and the electrode of the capacitive touch sensor (52a, 52b) with respect to the ground.

When the capacitance changes and the change amount exceeds a threshold value, the capacitive touch sensor module 50 detects a change to be analyzed. Sensitivity (ΔC/C) is used to indicate the amount of change in capacitance. The analysis of the composition and distribution of the capacitance helps to find a way to optimize the sensitivity in the measurement. The readings of each capacitive touch sensor (52a, 52b), originally used to read finger pulses on data screens (on mobiles, for example), is used in this application to detect the capacitances related to contact with the animal. The capacitances Cₜᵣ, C_{w}, Cₑ and C_{c} are collectively referred to as the parasitic capacitance Cₚ (in conditions without physical contact of the measuring terminal and the skin/hair of the dog 6).

The capacitance Cₜ is the total change in the capacitance (ΔC) of the system when a respiratory event occurs in the chest of the dog 6, said change produced in the capacitance (ΔC=Cₜ) being detected by the capacitive touch sensor module 50. The lower the parasitic capacitance Cₚ and the higher the Cₜ, the more easily breathing can be detected, i.e., the greater the sensitivity of system 1. For an example in which the capacitance Cₜ has a same value of 5pF, if the parasitic capacitance Cₚ is quantified at 100pF, the change of capacitance (AC=Ct) represents 5% of the parasitic capacitance Cₚ, while if the parasitic capacitance Cₚ is 20pF, the change of capacitance (ΔC=Cₜ) represents 25% of the parasitic capacitance Cₚ. Therefore, reducing the parasitic capacity Cₚ to the maximum in the capture system is necessary to increase the sensitivity of the electrical capacity measurements of the system, so a shielded connection between the capacitive touch sensor module 50 (integrated into a PCB 15) and the electrode (4a, 4b) installed in the harness 2 itself is preferably used.

The electrode (4a, 4b) is preferably implemented by a copper foil. In one embodiment, the support base (overlay) of the electrode (4a, 4b) itself is flexible, slightly elastic, with a plastic base that allows it to be water resistant and with a very low parasitic capacity. The thickness of the copper foil, which acts as the armature of the capacitor, is selected to allow capacities close to 2000 pF to be obtained. The support base and thickness contribute to obtaining a good sensitivity of the measurement system.

Figure 12 explains the operation of each capacitive touch sensor (52a, 52b). An internal current source of the capacitive touch sensor module 50 periodically charges and discharges each capacitive touch channel (53a, 53b). During charging and discharging, the voltage VP of the touch pin or lead (power lead of the capacitive touch sensor) oscillates between a high reference voltage DREFH and a low reference voltage DREFL. The charge is completed when the voltage reaches DREFH and the discharge is considered finished when the voltage reaches DREFL. A charge/discharge cycle is counted after completing the charge from DREFL to DREFH and the discharge back to DREFL. During each DREFH-DREFL-DREFH oscillation, the capacitive touch sensor (52a, 52b) generates an output pulse 55 that will be counted by the capacitive touch sensor module 50. The capacitance in the touch lead remains constant in an ideal environment without interference. The number of output pulses 55 is constant in the same time interval. The speed of loading and unloading of the touch pin is determined by the intensity of the current output from the internal current source. The intensity of the current source is reflected in the slope m of the voltage signal V_{P} of the touch lead.

When the chest of the dog 6 approaches the capacitive touch sensor (52a, 52b), a capacitor is formed between the sensor metal sheet (electrode 4a/4b) of the harness 2 and the chest of the animal, and its equivalent capacitance Cₜ is connected in parallel to the touch channel (53a, 53b), increasing the capacitance in the touch lead. According to the *formula* $I = C \frac{dV}{dt}$*,* if the charging/discharging current *I* remains the same and the capacitance *C* increases, the charging/discharging time will increase, and the output pulse count 55 will decrease during the same time interval. By comparing the difference between the output pulse counts 55 during the same time interval, it can be concluded if the capacitive touch sensor pad has been touched and at what level, being able to obtain a measurement of the variation of the capacitance proportional to the movement captured from the chest of the dog 6. In this way, by analyzing the ups and downs of said capacitance variation, the respiratory rate of the dog 6 can be detected.

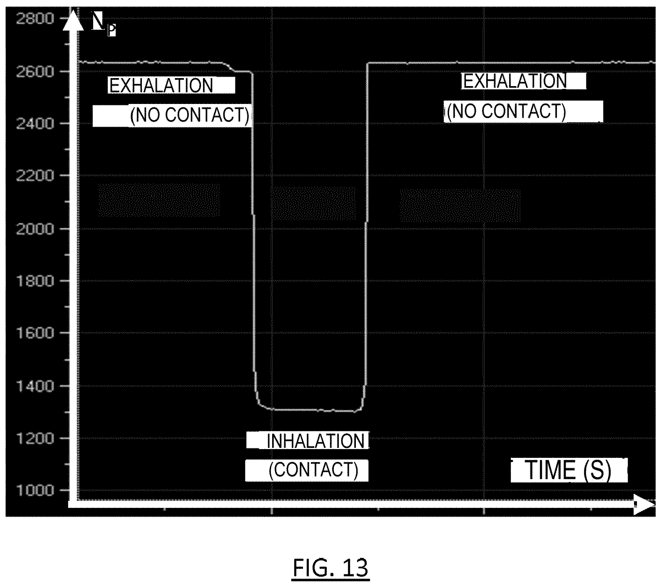

In the graph of figure 13 you can see the difference between there being no contact between harness 2 and the animal's chest ("NO CONTACT") and when there is actually contact ("CONTACT"). The vertical axis shows the counts of the output pulse counter 55 (number of pulses Np), which increase when the capacitance decreases (that is, when the chest of the dog 6 is separated from the electrode (4a, 4b) of the harness 2, or the harness 2 is not placed on the animal), and which decrease when the capacitance increases when the chest approaches the electrode (4a, 4b) of the harness 2; moreover, when the value of the number of pulses NP is lower, the result obtained will be more reliable. In summary, by counting the number of times the capacity increases/decreases per minute, the rate of breathing can be estimated. The capacitive touch sensor module 50 provides a number of counts proportional to the rate of breathing.

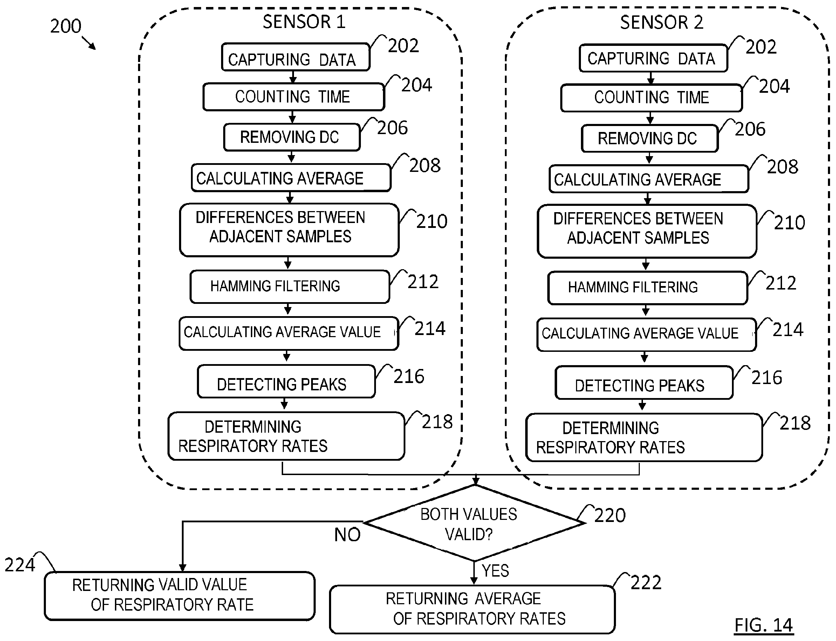

Figure 14 illustrates, in accordance with one embodiment, a flow chart of a process 200 of calculating the respiratory rate 40 from the acquisition of measurements, in which two capacitive sensors are employed (e.g., one for the left side of the thorax of the dog 6 and one for the right side). This process 200 for calculating the respiratory rate 40 comprises the following steps, for each of the sensors:
- Capturing data 202 by each capacitive sensor. The data processing unit 14 may be programmed to capture a given number N of samples.
- Counting time 204, to determine the sampling time T employed in the data capture 202.
- Removing the continuous component 206 (the average value, DC value) from the samples to use smaller values.
- Calculating the average 208 (e.g. of four consecutive points) to filter the signal.
- Calculating the difference 210 between each sample and the next.
- Applying a Hamming filter 212.
- Calculating the average value 214 to detect whether or not the harness 2 (and the measuring device 3) is placed on the dog 6. If the average value does not exceed a threshold value C_{MIN}, the captured data is discarded.
- Detecting 216 valid peaks (or valleys).
- Determining 218 the respiratory rate by calculating the average temporal distance 36 between peaks.

The process 200 of calculating the respiratory rate 40 also comprises checking 220 whether both respiratory rates (obtained for the first capacitive sensor and for the second capacitive sensor) are valid, that is, whether they are within a permitted range (e.g. 10-40 breaths per minute), in which case the average of both respiratory rate values is returned 222. If any of the values is invalid, only the valid respiratory rate value is returned 224.

## Claims

1. A system for measuring respiratory rate in dogs, comprising:
a dog harness (2) including at least one electrode (4a, 4b) arranged in a chest region, each electrode (4a, 4b) being electrically connected to a respective metal contact (7a, 7b);
a measuring device (3) comprising:
a housing (9);
a fastening system (10) configured to fasten the housing (9) to the harness (2);
at least one capacitive sensor (12a, 12b) configured to make electrical capacity measurements, each capacitive sensor (12a, 12b) being electrically connected to a corresponding metal contact (7a, 7b) of the harness (2) when the measuring device (3) is fastened to the harness (2);
at least one battery (13);
a data processing unit (14) configured for:
receiving an electrical capacity signal (31a, 31b) from each capacitive sensor (12a, 12b), where each electrical capacity signal (31a, 31b) includes a plurality of electrical capacity measurements performed by the corresponding capacitive sensor (12a, 12b) during a sampling time (T) when the harness (2) is placed on a dog (6);
filtering each electrical capacity signal (31a, 31b);
detecting local peaks (35) or valleys (37) in each filtered electrical capacity signal (33a, 33b);
calculating an average temporal distance (36) between the detected peaks (35) or valleys (37);
determining a respiratory rate associated with each filtered electrical capacity signal (33a, 33b) as a function of the corresponding average temporal distance (36) between peaks (35) or valleys (37); and
determining a respiratory rate (40) of the dog (6) from the respiratory rate associated with each filtered electrical capacity signal (33a, 33b).

2. The system according to claim 1, wherein the harness (2) includes two electrodes (4a, 4b) arranged on either side of the chest region.

3. The system according to claim 2, wherein the electrodes (4a, 4b) are curved metal sheets configured to contact both sides of the thorax of the dog (6).

4. The system according to any one of the preceding claims, wherein each capacitive sensor (12a, 12b) is a capacitive touch sensor (52a, 52b).

5. The system according to claim 4, comprising a capacitive touch sensor module (50), and wherein the connection between the capacitive touch sensor module (50) and the at least one electrode (4a, 4b) is shielded.

6. The system according to any one of claims 1 to 5, wherein the measuring device (3) comprises cables (16a, 16b) external to the housing (9) with respective connectors (26a, 26b) at their ends adapted to engage and contact the metal contacts (7a, 7b) of the harness (2).

7. The system according to any one of claims 1 to 5, wherein the measuring device (3) comprises connectors at the base of the housing (9) adapted to engage and contact the metal contacts (7a, 7b) of the harness (2).

8. The system according to any one of the preceding claims, wherein the data processing unit (14) is configured to discard a set of electrical capacity measurements whose average value does not exceed a threshold value (C_{MIN}).

9. The system according to any one of the preceding claims, wherein the data processing unit (14) is configured to discard detected peaks (35) or valleys (37) whose temporal distance to adjacent peaks (35) or valleys (37) does not exceed a minimum distance (D_{MIN}).

10. A method for measuring respiratory rate in dogs, comprising:
placing (102) on a dog (6) a harness (2) including at least one electrode (4a, 4b) arranged in a chest region, each electrode (4a, 4b) being electrically connected to a respective metal contact (7a, 7b);
fastening (104) to the harness (2) a measuring device (3) including at least one capacitive sensor (12a, 12b), such that each capacitive sensor (12a, 12b) is electrically connected to a corresponding metal contact (7a, 7b) of the harness (2);
receiving (106) an electrical capacity signal (31a, 31b) from each capacitive sensor (12a, 12b), where each electrical capacity signal (31a, 31b) includes a plurality of electrical capacity measurements performed by the corresponding capacitive sensor (12a, 12b) during a sampling time(T);
filtering (108) each electrical capacity signal (31a, 31b);
detecting (110) local peaks (35) or valleys (37) in each filtered electrical capacity signal (33a, 33b);
calculating (112) an average temporal distance (36) between the detected peaks (35) or valleys (37);
determining (114) a respiratory rate associated with each filtered electrical capacity signal (33a, 33b) as a function of the corresponding average temporal distance (36) between peaks (35) or valleys (37); and
determining (116) a respiratory rate (40) of the dog (6) from the respiratory rate associated with each filtered electrical capacity signal (33a, 33b).

11. The method according to claim 10, comprising discarding a set of electrical capacity measurements whose average value does not exceed a threshold value (C_{MIN}).

12. The method according to any one of claims 10 to 11, comprising discarding detected peaks (35) or valleys (37) whose temporal distance to adjacent peaks (35) or valleys (37) does not exceed a minimum distance (D_{MIN}).

## Amended claims

### Amended claims in accordance with Rule 137(2) EPC.

1. A system for measuring respiratory rate in dogs, comprising:
a dog harness (2) including at least one electrode (4a, 4b) arranged in a chest region, each electrode (4a, 4b) being a metal sheet electrically connected to a respective metal contact (7a, 7b) and arranged to be in contact with the skin of a dog (6) when the harness (2) is placed on the dog (6);
a measuring device (3) comprising:
a housing (9);
a fastening system (10) configured to fasten the housing (9) to the harness (2);
at least one capacitive touch sensor (52a, 52b) configured to make electrical capacity measurements, each capacitive touch sensor (52a, 52b) being electrically connected to a corresponding metal contact (7a, 7b) of the harness (2) when the measuring device (3) is fastened to the harness (2);
at least one battery (13);
a data processing unit (14) configured for:
receiving an electrical capacity signal (31a, 31b) from each capacitive touch sensor (52a, 52b), where each electrical capacity signal (31a, 31b) includes a plurality of electrical capacity measurements performed by the corresponding capacitive touch sensor (52a, 52b) during a sampling time (T) when the harness (2) is placed on the dog (6);
filtering each electrical capacity signal (31a, 31b);
detecting local peaks (35) or valleys (37) in each filtered electrical capacity signal (33a, 33b);
calculating an average temporal distance (36) between the detected peaks (35) or valleys (37);
determining a respiratory rate associated with each filtered electrical capacity signal (33a, 33b) as a function of the corresponding average temporal distance (36) between peaks (35) or valleys (37); and
determining a respiratory rate (40) of the dog (6) from the respiratory rate associated with each filtered electrical capacity signal (33a, 33b).

2. The system according to claim 1, wherein the harness (2) includes two electrodes (4a, 4b) arranged on either side of the chest region.

3. The system according to claim 2, wherein the electrodes (4a, 4b) are curved metal sheets configured to contact both sides of the thorax of the dog (6).

4. The system according to any one of the preceding claims, comprising a capacitive touch sensor module (50), and wherein the connection between the capacitive touch sensor module (50) and the at least one electrode (4a, 4b) is shielded.

5. The system according to any one of claims 1 to 4, wherein the measuring device (3) comprises cables (16a, 16b) external to the housing (9) with respective connectors (26a, 26b) at their ends adapted to engage and contact the metal contacts (7a, 7b) of the harness (2).

6. The system according to any one of claims 1 to 4, wherein the measuring device (3) comprises connectors at the base of the housing (9) adapted to engage and contact the metal contacts (7a, 7b) of the harness (2).

7. The system according to any one of the preceding claims, wherein the data processing unit (14) is configured to discard a set of electrical capacity measurements whose average value does not exceed a threshold value (C_{MIN}).

8. The system according to any one of the preceding claims, wherein the data processing unit (14) is configured to discard detected peaks (35) or valleys (37) whose temporal distance to adjacent peaks (35) or valleys (37) does not exceed a minimum distance (D_{MIN}).

9. A method for measuring respiratory rate in dogs, comprising:
placing (102) on a dog (6) a harness (2) including at least one electrode (4a, 4b) arranged in a chest region, each electrode (4a, 4b) being a metal sheet electrically connected to a respective metal contact (7a, 7b) and arranged to be in contact with the skin of a dog (6);
fastening (104) to the harness (2) a measuring device (3) including at least one capacitive touch sensor (52a, 52b), such that each capacitive touch sensor (52a, 52b) is electrically connected to a corresponding metal contact (7a, 7b) of the harness (2);
receiving (106) an electrical capacity signal (31a, 31b) from each capacitive touch sensor (52a, 52b), where each electrical capacity signal (31a, 31b) includes a plurality of electrical capacity measurements performed by the corresponding capacitive touch sensor (52a, 52b) during a sampling time(T);
filtering (108) each electrical capacity signal (31a, 31b);
detecting (110) local peaks (35) or valleys (37) in each filtered electrical capacity signal (33a, 33b);
calculating (112) an average temporal distance (36) between the detected peaks (35) or valleys (37);
determining (114) a respiratory rate associated with each filtered electrical capacity signal (33a, 33b) as a function of the corresponding average temporal distance (36) between peaks (35) or valleys (37); and
determining (116) a respiratory rate (40) of the dog (6) from the respiratory rate associated with each filtered electrical capacity signal (33a, 33b).

10. The method according to claim 9, comprising discarding a set of electrical capacity measurements whose average value does not exceed a threshold value (C_{MIN}).

11. The method according to any one of claims 9 to 10, comprising discarding detected peaks (35) or valleys (37) whose temporal distance to adjacent peaks (35) or valleys (37) does not exceed a minimum distance (D_{MIN}).
